(19) Europäisches Patentamt / European Patent Office / Office européen des brevets

(11) **EP 2 331 650 B1**

(12) **FASCICULE DE BREVET EUROPEEN**

(45) Date de publication et mention de la délivrance du brevet:
**20.09.2017 Bulletin 2017/38**

(21) Numéro de dépôt: **09807917.1**

(22) Date de dépôt: **07.07.2009**

(51) Int Cl.:
*C09K 3/14* *(2006.01)*     *C09G 1/02* *(2006.01)*
*C01F 17/00* *(2006.01)*     *A61K 8/02* *(2006.01)*
*A61K 8/19* *(2006.01)*     *B82Y 30/00* *(2011.01)*
*A61Q 17/04* *(2006.01)*     *B24B 37/04* *(2012.01)*

(86) Numéro de dépôt international:
**PCT/EP2009/058571**

(87) Numéro de publication internationale:
**WO 2010/020466 (25.02.2010 Gazette 2010/08)**

(54) **SUSPENSION LIQUIDE ET POUDRE DE PARTICULES D'OXYDE DE CERIUM, PROCEDES DE PREPARATION DE CELLES-CI ET UTILISATION DANS LE POLISSAGE**

FLÜSSIGE SUSPENSION UND PULVER AUS CEROXIDPARTIKELN, VERFAHREN ZU IHRER HERSTELLUNG UND VERWENDUNG FÜR POLIERVERFAHREN

LIQUID SUSPENSION AND POWDER OF CERIUM OXIDE PARTICLES, METHODS FOR MAKING THE SAME AND USES THEREOF IN POLISHING

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **22.08.2008 FR 0804676**

(43) Date de publication de la demande:
**15.06.2011 Bulletin 2011/24**

(73) Titulaire: **Rhodia Opérations**
**93306 Aubervilliers (FR)**

(72) Inventeurs:
• **CRINIERE, Guillaume**
**1400 NIVELLES (BE)**
• **THIERS, Laurent**
**F-91600 Savigny-sur-Orge (FR)**

(74) Mandataire: **Senninger, Thierry et al**
**Rhodia Operations**
**Département de la Propriété Industrielle**
**40, rue de la Haie-Coq**
**93306 Aubervilliers Cedex (FR)**

(56) Documents cités:
**EP-A- 1 234 801     WO-A-2008/043703**

**Description**

La présente invention concerne une suspension liquide et une poudre de particules d'oxyde de cérium, des procédés de préparation de celles-ci et leur utilisation dans le polissage notamment.

Le développement de l'industrie de l'électronique nécessite l'utilisation de plus en plus importante de compositions pour le polissage de diverses pièces comme les disques ou les composés diélectriques. Ces compositions se présentent sous la forme de suspensions et elles doivent répondre à un certain nombre de caractéristiques. Par exemple, elles doivent offrir un taux d'enlèvement de matière élevé qui traduit leur capacité abrasive. Elles doivent aussi présenter une défectivité la plus basse possible, on entend par défectivité notamment le taux de rayures que présente le substrat une fois traité par la composition.

Pour des raisons de stabilité et de facilité d'utilisation, ces suspensions doivent être constituées de particules de dimension submicronique, c'est à dire généralement inférieure à 300 nm. En outre, la présence de particules trop fines dans ces suspensions diminue leurs capacités abrasives. Par ailleurs, des particules trop grosses peuvent contribuer à une augmentation de la défectivité. On a donc besoin de suspensions dont les particules soient monodisperses. Il faut en outre noter que pour obtenir des performances optimales cette monodispersité doit s'appliquer à la fois aux particules primaires et aux particules secondaires, c'est-à-dire les agrégats constitués par les particules primaires.

Enfin, il peut être intéressant pour l'amélioration des capacités de polissage de disposer de suspensions pour lesquelles les particules à taille identiques par rapport à celles d'autres suspensions présentent néanmoins une surface spécifique plus importante.

On comprend donc que la mise au point de ces suspensions est un problème complexe.

EP 1234801 décrit un procédé de préparation d'oxyde de cérium qui est différent du procédé de l'invention car il comprend une calcination d carbonate de cérium et un broyage humide.

WO 2008/043703 décrit un procédé de préparation de dispersions de particules monodisperses qui semble proche de celui revendiqué mais qui présente toutefois une différence importante car il ne met pas en oeuvre une dispersion colloïdale de cérium IV comme produit de départ. La présente demande comporte un exemple comparatif selon l'enseignement de WO 2008/043703.

L'objet de l'invention est un procédé qui permet de fournir des suspensions répondant aux conditions décrites ci-dessus, c'est à dire des suspensions dont les particules sont monodisperses et, éventuellement à surface spécifique améliorée.

Dans ce but, le procédé permet de préparer une suspension de particules d'oxyde de cérium dans une phase liquide, ces particules (particules secondaires) présentant une taille moyenne d'au plus 200 nm et étant constituées de particules primaires dont les tailles mesurées par MET présentent une valeur moyenne d'au plus 150 nm avec un écart type dont la valeur est d'au plus 30% de la valeur de ladite taille moyenne et pour lesquelles le rapport de la taille moyenne mesurée par MET à la taille moyenne mesurée par BET est d'au moins 1,5.

D'autres caractéristiques, détails et avantages de l'invention apparaîtront encore plus complètement à la lecture de la description qui va suivre, des exemples concrets mais non limitatifs destinés à l'illustrer et du dessin annexé dans lequel :

- la figure 1 est une photo MET d'une suspension selon l'invention;
- la figure 2 est une photo MET d'une suspension selon l'art antérieur.

Pour la suite de la description, l'expression « suspension de particules d'oxyde de cérium » désigne un système constitué de fines particules solides de dimension submicronique à base de cet oxyde, dispersées de manière stable dans une phase liquide, lesdites particules pouvant éventuellement, en outre, contenir des quantités résiduelles d'ions liés ou adsorbés tels que par exemple des nitrates ou des ammoniums.

Toujours pour la suite de la description, on entend par surface spécifique, la surface spécifique B.E.T. déterminée par adsorption d'azote conformément à la norme ASTM D 3663-78 établie à partir de la méthode BRUNAUER - EMMETT- TELLER décrite dans le périodique "The Journal of the American Chemical Society, 60, 309 (1938)". Enfin, par MET, on entend microscopie électronique à transmission.

Les particules de la suspension sont à base d'oxyde de cérium qui est généralement de l'oxyde cérique cristallisé.

Les particules qui constituent la suspension présentent une taille moyenne d'au plus 200 nm sont appelées dans la suite de la description « particules secondaires ». Ces particules sont des agrégats d'autres particules plus fines, agrégées, appelées par la suite « particules primaires ». Selon une caractéristique importante de l'invention, ces particules primaires sont fines et monodisperses. Elles présentent en effet une taille moyenne mesurée par MET d'au plus 150 nm avec un écart type dont la valeur est d'au plus 30% de la valeur de la taille moyenne de ces particules primaires.

L'écart type mentionné dans la présente invention et mesuré aussi en mettant en oeuvre la technique MET a le sens habituel mathématique, il s'agit de la racine carrée de la variance et il s'exprime par la formule :

$$\sigma = \sqrt{\frac{1}{n}\sum_{i=1}^{n}(x_i - \overline{x})^2}$$

n étant le nombre de particules pris en compte dans la mesure,

$x_i$ étant la taille d'une particule i

$\overline{x}$ étant la valeur moyenne de la taille des particules $(1/n\sum_i x_i)$

La mesure de la taille des n différentes particules est faite à partir d'un cliché obtenu par MET. Cet écart type peut être de préférence d'au plus 20%, plus particulièrement d'au plus 15%, de la valeur de la taille moyenne des particules primaires.

Les particules primaires peuvent plus particulièrement avoir des tailles qui présentent une valeur moyenne d'au plus 130 nm. Ces tailles moyennes de particules primaires peuvent être par ailleurs d'au moins 50 nm, notamment d'au moins 80 nm et plus particulièrement d'au moins 100 nm.

Une autre caractéristique des particules primaires de la suspension selon l'invention est le rapport de leur taille moyenne mesurée selon différentes techniques.

Plus précisément, et comme indiqué plus haut, le rapport de la taille moyenne mesurée par MET à la taille moyenne mesurée par BET est d'au moins 1,5. Ce rapport peut être d'au moins 2. Par taille moyenne mesurée par BET on entend la taille théorique obtenue à partir de la valeur de surface spécifique mesurée par la technique BET et en supposant une particule sphérique non poreuse de $CeO_2$ de densité 7,2.

Sans vouloir être lié par une théorie, le fait que le rapport des tailles soit différent de 1 peut traduire des défauts de surface dans les particules, la surface de celles-ci apparaissant moins bien facettée que dans des produits connus. Ces défauts peuvent induire une augmentation de la surface spécifique des particules.

Comme indiqué plus haut, les particules primaires forment des agrégats qui constituent ainsi les particules secondaires. Ces particules secondaires peuvent avoir plus particulièrement une taille moyenne d'au plus 150 nm, plus particulièrement d'au plus 120 nm et encore plus particulièrement d'au plus 100 nm.

Par ailleurs, selon une autre caractéristique avantageuse de l'invention, ces particules secondaires sont elles aussi monodisperses. Elles peuvent présenter en effet un indice de dispersion d'au plus 0,5. Cet indice peut être notamment d'au plus 0,4, plus particulièrement d'au plus 0,3 et encore plus particulièrement d'au plus 0,2.

Pour l'ensemble de la description concernant les particules secondaires, la taille moyenne et l'indice de dispersion sont les valeurs obtenues en mettant en oeuvre la technique de diffraction laser en utilisant un granulomètre laser (répartition en volume).

On entend par indice de dispersion le rapport :

$$\sigma/m = (d_{90}-d_{10})/2d_{50}$$

dans lequel :

- $d_{90}$ est la taille ou diamètre des particules pour lequel 90% des particules ont un diamètre inférieur à $d_{90}$;
- $d_{10}$ est la taille ou diamètre des particules pour lequel 10% des particules ont un diamètre inférieur à $d_{10}$;
- $d_{50}$ est la taille ou diamètre moyen des particules.

La phase liquide des suspensions selon l'invention peut être de différente nature. Ce peut être tout d'abord l'eau. Ce peut être aussi un mélange eau/solvant miscible à l'eau. On peut citer comme exemple de solvant de ce type, les alcools comme le méthanol ou l'éthanol, les glycols comme l'éthylène glycol, les dérivés acétates des glycols comme le monoacétate d'éthylène glycol ou les polyols. La phase liquide peut enfin être constituée par un solvant organique.

Comme exemple de solvant organique, on peut citer les hydrocarbures aliphatiques comme l'hexane, l'heptane, l'octane, le nonane, les hydrocarbures cycloaliphatiques inertes tels que le cyclohexane, le cyclopentane, le cycloheptane, les hydrocarbures aromatiques tels que le benzène, le toluène, l'éthylbenzène, les xylènes, les naphtènes liquides. Conviennent également les coupes pétrolières du type Isopar ou Solvesso (marques déposées par la Société EXXON), notamment Solvesso 100 qui contient essentiellement un mélange de méthyléthyl- et triméthyl-benzène, le Solvesso 150 qui renferme un mélange d'alcoylbenzènes en particulier de diméthylbenzène et de tétraméthylbenzène et l'Isopar qui contient essentiellement des hydrocarbures iso- et cyclo-paraffiniques en C-11 et C-12. On peut aussi citer, comme autres coupes pétrolières, celles de type Petrolink® de la société Petrolink ou de type Isane® de la société Total.

On peut mettre en oeuvre également comme solvant organique des hydrocarbures chlorés tels que le chloro- ou le dichloro-benzène, le chlorotoluène. Les éthers ainsi que les cétones aliphatiques et cycloaliphatiques comme par exemple l'éther de diisopropyle, l'éther de dibutyle, la méthyléthylcétone, la méthylisobutylcétone, la diisobutylcétone, l'oxyde de mésityle, peuvent être envisagés.

Des esters peuvent être utilisés comme ceux issus de la réaction d'acides avec des alcools en C1 à C8 et notamment les palmitates d'alcools secondaires tel l'isopropanol. On peut mentionner l'acétate de butyle à titre d'exemple.

Bien entendu, la phase liquide peut être à base d'un mélange de deux ou plusieurs hydrocarbures ou composés du type décrit ci-dessus.

Les suspensions présentent une teneur globale en oxyde, c'est-à-dire en oxyde de cérium qui peut varier dans de larges limites et qui peut être par exemple d'au plus 40%, notamment d'au plus 25% à 30% en masse d'oxyde.

De même, le pH de ces suspensions peut être compris dans une large gamme. Ainsi, le pH des suspensions issues des procédés de préparation qui seront décrits plus loin est généralement compris entre 2 et 6, plus particulièrement entre 2 et 5, les suspensions restant stables, au sens donné ci-dessous, dans cette gamme de pH. Toutefois, la stabilité peut être améliorée soit à l'intérieur de ces gammes de pH soit au-delà de la valeur de 5 ou de 6 d'une manière connue, par addition à la suspension de composés comme des polymères ou molécules anioniques ou zwiterionic. Comme composés de ce type on peut citer ceux obtenu par la polymérisation d'au moins un monomère choisi parmi les entités suivantes: un acide ou un anhydride monocarboxylique ou polycarboxylique, linéaire ou ramifié, aliphatique, cyclique ou aromatique, à insaturation éthylénique. A titre d'exemple on peut citer l'acide polyacrylique ou l'acide citrique.

On notera enfin que les suspensions de l'invention sont stables. On entend par là que l'on n'observe pas sur ces suspensions de formation d'un gâteau de décantation avant plusieurs jours, par exemple au moins 8 jours. De plus le gâteau de décantation, s'il se forme, peut être remis en suspension par simple agitation.

La suspension peut être préparée par un procédé qui comprend les étapes suivantes :

- (a) on prépare une solution d'un sel de cérium III qui comprend en outre une dispersion colloïdale de cérium IV;
- (b) on met en contact sous atmosphère inerte cette solution avec une base ce par quoi on obtient un précipité;
- (c) on soumet le milieu obtenu à l'étape précédente à un traitement thermique sous atmosphère inerte, au moins une des étapes (a), (b) ou (c) étant conduite en présence d'ions nitrates;
- (d) on effectue successivement mais dans un ordre quelconque une acidification et un lavage du milieu ainsi obtenu ce par quoi on obtient la suspension.

La première étape (a) du procédé ci-dessus consiste donc à préparer une solution de départ qui est une solution d'un sel de cérium III. Comme sels de cérium III, on peut utiliser plus particulièrement le nitrate, le chlorure, le sulfate ou le carbonate de cérium III ainsi que des mélanges de ces sels comme des mixtes nitrate/chlorure. D'une manière connue cette solution de départ doit présenter l'acidité convenable pour que le cérium soit bien entièrement présent en solution.

La solution de départ comprend en outre du cérium IV. Ce cérium IV est apporté par une dispersion colloïdale de cet élément. On entend par dispersion colloïdale de cérium IV tout système constitué de fines particules solides de dimensions colloïdales à base notamment de composés de type oxyde et/ou oxyde hydraté (hydroxyde) du cérium, en suspension dans une phase liquide aqueuse, lesdites particules pouvant en outre, éventuellement, contenir des ions liés ou adsorbés tels que par exemple des nitrates et des ammoniums.

On utilise de préférence des dispersions dont la taille de particules est d'au plus 20 nm, plus particulièrement d'au plus 10 nm. Les tailles données ici doivent être entendues comme désignant le diamètre hydrodynamique moyen des particules, tel que déterminé sur la dispersion par diffusion quasi-élastique de la lumière (DQEL) selon la méthode décrite par Michael L. Mc CONNELL dans la revue Analytical Chemistry 53, n° 8, 1007 A, (1981). De telles dispersions sont bien connues, on peut citer notamment celles décrites dans EP 206906 A1, EP 208580 A1, EP 208581 A1, EP 433133 A1 ou EP 1265815 A1.

Généralement, la quantité de cérium IV est telle que le rapport molaire (Ce IV/Ce total) dans la solution de départ soit compris entre 1/5.000 et 1/500, plus particulièrement entre 1/4.000 et 1/2.000.

La solution de départ préparée à l'étape (a) peut être préalablement dégazée par bullage d'un gaz inerte. Par « gaz inerte » ou « atmosphère inerte », on entend pour la présente description une atmosphère ou un gaz exempt d'oxygène, le gaz pouvant être par exemple de l'azote ou de l'argon.

La seconde étape (b) du procédé consiste à faire réagir la solution de départ avec une base. Comme base, on peut utiliser notamment les produits du type hydroxyde. On peut citer les hydroxydes d'alcalins ou d'alcalino-terreux et l'ammoniaque. On peut aussi utiliser les amines secondaires, tertiaires ou quaternaires. Toutefois, les amines et l'ammoniaque peuvent être préférés dans la mesure où ils diminuent les risques de pollution par les cations alcalins ou alcalino terreux. La base peut aussi être préalablement dégazée par bullage d'un gaz inerte.

Pour conduire la réaction de la seconde étape du procédé, la mise en contact peut se faire dans un ordre quelconque d'introduction des réactifs. Toutefois, il est préférable d'introduire la solution de départ dans un milieu contenant la base. Cette seconde étape doit être conduite sous atmosphère inerte, soit dans un réacteur fermé soit dans un réacteur semi-fermé avec balayage par le gaz inerte. La mise en contact se fait généralement dans un réacteur agité. Enfin, cette seconde étape est généralement effectuée à température ambiante (20°C-25°C) ou à une température d'au plus 50°C.

La troisième étape (c) du procédé est un traitement thermique du milieu réactionnel obtenu à l'issue de l'étape précédente. Ce traitement consiste à chauffer le milieu et à le maintenir à une température qui est généralement d'au plus 95°C et plus particulièrement comprise entre 60°C et 95°C. La durée de ce traitement peut être comprise entre quelques minutes et quelques heures. Ce traitement est effectué aussi sous atmosphère inerte, ce qui a été décrit au sujet de cette atmosphère pour la seconde étape s'appliquant de même ici. De même le traitement se fait dans un réacteur agité.

Selon une caractéristique du procédé de l'invention, au moins une des étapes (a), (b) ou (c) doit être conduite en présence d'ions nitrates. Ces ions nitrates peuvent être apportés par addition d'acide nitrique, plus particulièrement à l'étape (a), lors de la préparation de la solution de cérium III ou encore par l'utilisation, à l'étape (a), d'un nitrate de cérium comme sel de cérium III. La quantité d'ions nitrates, exprimée par le rapport molaire $NO_3^-/Ce^{3+}$ est généralement comprise entre 1/3 et 5.

La dernière étape du procédé, étape (d), comprend en fait deux opérations successives qui peuvent être effectuées dans un ordre quelconque. Ces opérations sont d'une part une acidification et d'autre part un lavage. On va décrire ci-dessous plus précisément ces opérations dans le cas d'un enchaînement acidification puis lavage. L'acidification a lieu généralement après refroidissement du milieu obtenu à l'issue de l'étape (c) par addition d'un acide. On peut utiliser tout acide minéral ou organique. On utilise plus particulièrement l'acide nitrique. La quantité d'acide ajoutée est telle que le pH du milieu après acidification soit compris entre 2 et 5. Cette opération peut être conduite soit à l'air, soit sous atmosphère inerte.

L'acidification est suivie d'un lavage qui a pour but d'éliminer de la suspension les espèces solubles, essentiellement des sels. Le lavage peut se faire de différentes manières avec ou sans séparation solide/liquide.

On peut ainsi l'effectuer en séparant les particules solides de la phase liquide par exemple par filtration frontale, décantation ou centrifugation. Le solide obtenu est remis ensuite en suspension dans une phase aqueuse. On peut aussi procéder par filtration tangentielle.

Ce lavage peut être éventuellement renouvelé si nécessaire par exemple jusqu'à l'obtention d'une conductivité donnée de la suspension, la conductivité mesurant le taux d'impuretés présentes dans cette suspension.

Comme indiqué plus haut, l'ordre des opérations peut être inversé par rapport à ce qui vient d'être décrit. Ainsi, à l'issue de l'étape (c) et, là aussi, généralement après refroidissement du milieu obtenu, on peut alors procéder à un lavage de la manière décrite ci-dessus. A l'issue du lavage, on effectue ensuite l'acidification du milieu obtenu.

A l'issue des étapes qui viennent d'être décrites il est possible de traiter la suspension obtenue dans un appareil de désagglomération de type connu par exemple un appareil de traitement par ultra sons, de traitement par jet double impact ou un appareil de broyage humide.

On obtient à l'issue de l'étape (d) la suspension. Dans le cas d'une suspension partiellement ou totalement en milieu solvant organique différent de l'eau, cette suspension peut être préparée d'une manière connue en soi à partir d'une suspension aqueuse telle qu'obtenue par le procédé qui vient d'être décrit et par mise en contact avec le solvant organique.

A ce stade, il peut être avantageux d'ajouter dans la phase organique un agent promoteur dont la fonction est d'accélérer le transfert des particules de la phase aqueuse à la phase organique et d'améliorer la stabilité des suspensions organiques obtenues.

A titre d'agent promoteur, on peut utiliser les composés à fonction alcool et tout particulièrement des alcools aliphatiques linéaires ou ramifiés ayant de 6 à 12 atomes de carbone. Comme exemples spécifiques, on peut citer l'éthyl-2 hexanol, le décanol, le dodécanol ou leurs mélanges. La mise en contact peut se faire à température ambiante par exemple environ 20°C, mais aussi à une température plus élevée, par exemple dans un intervalle allant de 60°C à 150°C. La séparation entre les phases aqueuse et organique se fait par exemple par distillation, par décantation ou centrifugation en fonction de la nature du solvant organique.

Une poudre redispersible de particules d'oxyde de cérium peut être obtenue à partir d'une suspension selon l'invention par séchage puis calcination à une température qui peut être par exemple d'au plus 300°C et notamment comprise entre 100°C et 200°C, sur une durée qui peut varier entre quelques minutes et quelques heures.Cette poudre a pour caractéristique le fait qu'après introduction dans une phase liquide et redispersion dans une phase liquide elle conduit à une suspension selon l'invention telle que décrite plus haut. La redispersion se fait par simple agitation de la poudre dans la phase liquide.

Une suspension pour polissage peut comprendre soit une suspension telle que décrite plus haut, soit une suspension telle qu'obtenue par les procédés décrits précédemment soit encore une suspension obtenue après redispersion d'une poudre redispersible. Cette suspension peut être utilisée au polissage du verre, par exemple dans l'industrie de la cristallerie, de la glacerie, du verre plat, des écrans de télévision, des lunettes, ou encore au polissage des matières céramiques ou autres matériaux de type vitreux. Cette suspension peut aussi être utilisée tout particulièrement pour le polissage de type CMP dans l'industrie de l'électronique et donc pour le polissage des substrats métalliques entrant dans la constitution des microprocesseurs mais aussi pour le polissage des couches isolantes de ces mêmes microprocesseurs, la suspension de l'invention étant particulièrement adaptée au polissage de celles-ci. Ces couches sont généralement en silice (silice dopée, silice poreuse). Généralement, de telles suspensions comprennent, outre le composé à propriété abrasive comme les particules d'oxyde de cérium, des additifs comme un dispersant ou un oxydant.

Comme autres applications des suspensions, on peut citer la catalyse notamment pour post combustion automobile, dans ce cas les suspensions sont utilisées dans la préparation de catalyseurs. Les suspensions peuvent aussi être mises en oeuvre pour leurs propriétés anti-UV, par exemple dans la préparation de films de polymères (du type acrylique ou polycarbonate par exemple), de peintures, de papiers ou de compositions cosmétiques notamment dans la préparation de crèmes anti-UV.

Des exemples vont maintenant être donnés.

## EXEMPLE 1

Une solution de départ est préparée par addition de 830,6 g d'une solution de nitrate de cérium trivalent 2,88 M (d=1,715) et de 740 mg d'une solution colloïdale d'oxyde de cérium 0,70 M (d=1,115) dont la taille des particules mesurée par DQEL est de 6 nm. Cette solution, de rapport molaire $Ce^{4+}/Ce_{total}$ de 1/3000, est chargée dans un réservoir semi-fermé puis dégazée sous vive agitation et sous bullage d'azote pendant 2 heures.

Une solution d'ammoniaque diluée est préparée par ajout de 5041,0 g d'eau permutée et d'une solution de 423,9 g d'ammoniaque à 28%. Cette solution est chargée dans un réacteur double enveloppe de 6L semi fermé puis mise sous agitation et sous bullage d'azote pendant 2 heures.

La solution de départ préparée précédemment est ensuite ajoutée à température ambiante dans la solution d'ammoniaque diluée en 30 min, sous agitation et sous balayage d'azote.

Le mélange réactionnel est ensuite monté en température à 85°C en ½ d'heure puis maintenu à cette température pendant 4h toujours sous balayage d'azote.

A l'issue de ce traitement thermique, le mélange réactionnel est laissé à refroidir puis il est acidifié à pH de 4 par ajout d'acide nitrique 68%. Le balayage d'azote est arrêté et la suspension finalement lavée par centrifugation, élimination des eaux de centrifugation et remise en suspension du gâteau dans l'eau permutée. Plusieurs cycles de lavage par centrifugation sont effectués jusqu'à l'obtention d'une suspension finalement ajustée à 35% en poids en $CeO_2$ ayant un pH de 5 et une conductivité ionique de 0,2 mS/cm. La suspension est désagglomérée par passages successifs dans un homogénéiseur à jet double impact.

La suspension est observée en MET. Sur une photo de plusieurs centaines de particules représentatives de la suspension, on dénombre et on mesure chacune des particules, ce par quoi on obtient une taille moyenne de 125 nm avec un écart type de 24 nm, soit 19% de ladite taille moyenne.

Une partie de la suspension est séchée à l'étuve à 250°C, ce qui permet l'obtention d'une poudre de $CeO_2$. Le diffractogramme X de cette poudre a la signature de $CeO_2$ cristallisé (fiche ASTM 34-394).

La surface spécifique BET déterminée par adsorption d'azote est de 15 $m^2/g$, ce qui donne une taille moyenne des particules primaires de 55 nm.

Le rapport de la taille moyenne mesurée par MET à la taille moyenne mesurée par BET est de 2,2.

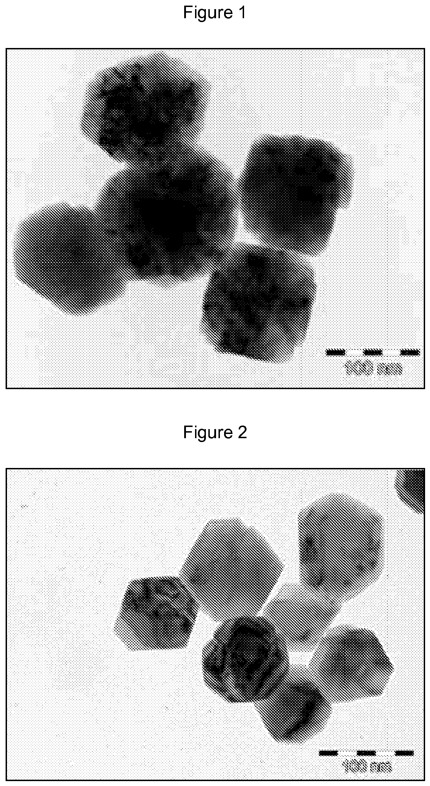

La taille des particules secondaires est mesurée à partir d'un granulomètre laser de type Horiba LA910 en considérant une valeur de l'indice optique de $CeO_2$ dans l'eau de 1,7. La taille médiane $d_{50}$ est de 115 nm. L'indice de dispersion $\sigma/m$ calculé à partir des valeurs $d_{10}$, $d_{50}$ et $d_{90}$ respectivement de 93, 115 et 146 nm est de 0,2. La figure 1 est une photo MET de la suspension obtenue dans l'exemple 1.

## EXEMPLE 2

La suspension telle qu'obtenue dans l'exemple 1 est diluée à 20% en poids en $CeO_2$ par ajout d'eau permutée. 100 g de cette suspension sont ajoutés sous agitation dans 100 g d'une solution d'acide polyacrylique (PAA) de masse molaire Mw=2000 g/mol diluée de telle sorte que le ratio $PAA/CeO_2$ final soit de 1,2% en poids. Le pH de la suspension ainsi obtenue est remonté à 8 par ajout d'ammoniaque 28%.

La taille des particules secondaires est mesurée à partir d'un granulomètre laser de type Horiba LA910 en considérant une valeur de l'indice optique de $CeO_2$ dans l'eau de 1,7. La taille médiane $d_{50}$ est de 112 nm. L'indice de dispersion $\sigma/m$ calculé à partir des valeurs $d_{10}$, $d_{50}$ et $d_{90}$ respectivement de 93, 112 et 140 nm est de 0,2.

## EXEMPLE COMPARATIF

Une solution de nitrate de cérium diluée est préparée par addition de 6,4 kg d'une solution de nitrate de cérium trivalent 2,88 M (d=1,715), de 1,0 kg d'une solution de $HNO_3$ 68%, de 4,8 kg d'eau permutée et de 8,87 g de nitrate de cérium tétravalent 1,39 M (d=1,440). Cette solution, de rapport molaire $Ce^{4+}/Ce_{total}$ de 1/1250, est chargée dans un réservoir semi-fermé puis dégazée sous vive agitation et sous bullage d'azote pendant 2 heures.

Une solution d'ammoniaque diluée est préparée par ajout de 22,6 kg d'eau permutée et d'une solution de 4,6 kg d'ammoniaque à 28%. Cette solution est chargée dans un réacteur double enveloppe de 40L semi fermé puis mise sous agitation et sous bullage d'azote pendant 2 heures.

La solution de nitrate de cérium diluée est ensuite ajoutée à température ambiante dans la solution d'ammoniaque diluée en 30 min. sous agitation et sous balayage d'azote.

Le mélange réactionnel est ensuite monté en température à 83°C en ½ d'heure puis maintenu à cette température pendant 4h toujours sous balayage d'azote.

A l'issue de ce traitement thermique, le mélange réactionnel est laissé à refroidir puis il est acidifié à pH de 2 par ajout d'acide nitrique 68%. Le balayage d'azote est arrêté et la suspension finalement lavée par centrifugation, élimination des eaux de centrifugation et remise en suspension du gâteau dans l'eau permutée. Plusieurs cycles de lavage par centrifugation sont effectués jusqu'à l'obtention d'une suspension finalement ajustée à 10% en poids en CeO$_2$ ayant un pH de 3,2 et une conductivité ionique de 0,38 mS/cm.

La suspension est observée en MET. Sur une photo de plusieurs centaines de particules représentatives de la suspension, on dénombre et on mesure chacune des particules, ce par quoi on obtient une taille moyenne de 65 nm avec un écart type de 5,9 nm, soit 9% de la dite taille moyenne.

Une partie de la suspension est séchée à l'étuve à 200°C, ce qui permet l'obtention d'une poudre de CeO$_2$. Le diffractogramme X de cette poudre a la signature de CeO$_2$ cristallisé (fiche ASTM 34-394).

La surface spécifique BET déterminée par adsorption d'azote est de 13 m$^2$/g, ce qui donne une taille moyenne des particules primaires de 64 nm.

Le rapport de la taille moyenne mesurée par MET à la taille moyenne mesurée par BET est de 1,0.

La taille des particules secondaires est mesurée à partir d'un granulomètre laser de type Horiba LA910 en considérant une valeur de l'indice optique de CeO$_2$ dans l'eau de 1,7. La taille médiane d$_{50}$ est de 112 nm. L'indice de dispersion σ/m calculé à partir des valeurs d$_{10}$, d$_{50}$ et d$_{90}$ respectivement de 84, 112 et 157 nm est de 0,32. La figure 2 est une photo MET de la suspension obtenue dans cet exemple comparatif.

## Revendications

1. Procédé de préparation d'une suspension de particules d'oxyde de cérium dans une phase liquide, ces particules (particules secondaires) présentant une taille moyenne d'au plus 200 nm, ces particules secondaires étant constituées de particules primaires dont les tailles mesurées par MET présentent une valeur moyenne d'au plus 150 nm avec un écart type dont la valeur est d'au plus 30% de la valeur de ladite taille moyenne et pour lesquelles le rapport de la taille moyenne mesurée par MET à la taille moyenne mesurée par BET est d'au moins 1,5; procédé qui comprend les étapes suivantes :

   - (a) on prépare une solution d'un sel de cérium III qui comprend en outre du cérium IV;
   - (b) on met en contact sous atmosphère inerte cette solution avec une base ce par quoi on obtient un précipité;
   - (c) on soumet le milieu obtenu à l'étape précédente à un traitement thermique sous atmosphère inerte, au moins une des étapes (a), (b) ou (c) étant conduite en présence d'ions nitrates;
   - (d) on effectue successivement mais dans un ordre quelconque une acidification et un lavage du milieu ainsi obtenu ce par quoi on obtient la suspension;

procédé **caractérisé en ce que** dans la solution de l'étape (a) le cérium IV est apporté sous forme d'une dispersion colloïdale de cet élément.

2. Procédé selon la revendication 1, **caractérisé en ce que** la solution d'un sel de cérium III de l'étape (a) contient du cérium IV dans une proportion molaire cérium IV/cérium total comprise entre 1/5.000 et 1/500, plus particulièrement entre 1/4.000 et 1/2.000.

3. Procédé selon la revendication 1 ou 2, **caractérisé en ce que** la teneur en ions nitrates lors de la mise en oeuvre au moins une des étapes (a), (b) ou (c) exprimée par le rapport molaire NO$_3^-$/Ce$^{3+}$ est comprise entre 1/3 et 5.

4. Procédé selon l'une des revendications 1 à 3, **caractérisé en ce qu'**on effectue le traitement thermique à une température d'au plus 95°C.

5. Procédé de préparation d'une suspension dans laquelle la phase liquide est un solvant organique, **caractérisé en ce que** l'on met en contact avec ledit organique une suspension telle qu'obtenue par le procédé selon l'une des revendications 1 à 4.

## Patentansprüche

1. Verfahren zur Herstellung einer Suspension von Ceroxidteilchen in einer flüssigen Phase, wobei diese Teilchen (Sekundärteilchen) eine mittlere Größe von höchstens 200 nm aufweisen, wobei die Sekundärteilchen aus Primärteilchen bestehen, deren mittels TEM gemessene Größen einen Mittelwert von höchstens 150 nm mit einer Standardabweichung, deren Wert höchstens 30% des Wertes der mittleren Größe beträgt, aufweisen und bei denen das Verhältnis der mittels TEM gemessenen mittleren Größe zu der mittels BET gemessenen mittleren Größe mindestens 1,5 beträgt; wobei das Verfahren die folgenden Schritte umfasst:

   - (a) man stellt eine Lösung eines Cer(III)-Salzes her, die außerdem Cer(IV) umfasst;
   - (b) man bringt diese Lösung unter einer inerten Atmosphäre mit einer Base in Kontakt, wodurch man einen Niederschlag erhält;
   - (c) man unterwirft das im vorhergehenden Schritt erhaltene Medium einer thermischen Behandlung unter einer inerten Atmosphäre, wobei mindestens einer der Schritte (a), (b) oder (c) in Gegenwart von Nitrationen durchgeführt wird;
   - (d) man führt nacheinander, jedoch in beliebiger Reihenfolge, eine Ansäuerung und Waschen des so erhaltenen Mediums durch, wodurch man die Suspension erhält,

wobei das Verfahren **dadurch gekennzeichnet ist, dass** in die Lösung von Schritt (a) das Cer(IV) in Form einer kolloidalen Dispersion dieses Elements eingebracht wird.

2. Verfahren nach Anspruch 1, **dadurch gekennzeichnet, dass** die Lösung eines Cer(III)-Salzes von Schritt (a) Cer(IV) in einem Molverhältnis von Cer(IV)/Gesamt-Cer zwischen 1/5000 bis 1/500, ganz besonders zwischen 1/4000 und 1/2000 umfasst.

3. Verfahren nach Anspruch 1 oder 2, **dadurch gekennzeichnet, dass** der Gehalt an Nitrationen bei der Durchführung mindestens eines der Schritte (a), (b) oder (c), ausgedrückt als das Molverhältnis $NO_3^-/Ce^{3+}$, zwischen 1/3 und 5 beträgt.

4. Verfahren nach einem der Ansprüche 1 bis 3, **dadurch gekennzeichnet, dass** man die thermische Behandlung bei einer Temperatur von höchstens 95°C durchführt.

5. Verfahren zur Herstellung einer Suspension, in der die flüssige Phase ein organisches Lösungsmittel ist, **dadurch gekennzeichnet, dass** man mit dem organischen eine durch das Verfahren nach einem der Ansprüche 1 bis 4 erhaltene Suspension in Kontakt bringt.

**Claims**

1. Process for preparing a suspension of cerium oxide particles in a liquid phase, these particles (secondary particles) having an average size of at most 200 nm, these secondary particles consisting of primary particles whose average size measured by TEM is of at most 150 nm with a standard deviation of at most 30% of the value of said average size, and for which the ratio of the average size measured by TEM to the average size measured by BET is at least 1.5; which process comprises the following steps:

- (a) a solution of a cerium III salt which also comprises cerium IV is prepared;
- (b) this solution is brought into contact with a base, under an inert atmosphere, by virtue of which procedure a precipitate is obtained;
- (c) the medium obtained in the preceding step is subjected to a thermal treatment under an inert atmosphere, at least one of steps (a), (b) or (c) being carried out in the presence of nitrate ions;
- (d) an acidification and a washing of the medium thus obtained are carried out successively but in any order, by virtue of which procedure the suspension is obtained;

process **characterized in that**, in the solution of step (a), the cerium IV is provided in the form of a colloidal dispersion of this element.

2. Process according to Claim 1, **characterized in that** the solution of a cerium III salt of step (a) contains cerium IV in a cerium IV/total cerium molar proportion of between 1/5000 and 1/500 more particularly between 1/4000 and 1/2000.

3. Process according to Claim 1 or 2, **characterized in that** the content of nitrate ions during the implementation of at least one of steps (a), (b) or (c), expressed by the $NO_3^-/Ce^{3+}$ molar ratio, is between 1/3 and 5.

4. Process according to one of Claims 1 to 3, **characterized in that** the thermal treatment is carried out at a temperature of at most 95°C.

5. Process for preparing a suspension in which the liquid phase is an organic solvent, **characterized in that** a suspension as obtained by means of the process according to one of Claims 1 to 4 is brought into contact with said organic.

Figure 1

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1234801 A
- WO 2008043703 A
- EP 206906 A1
- EP 208580 A1
- EP 208581 A1
- EP 433133 A1
- EP 1265815 A1

**Littérature non-brevet citée dans la description**

- *The Journal of the American Chemical Society,* 1938, vol. 60, 309
- **MICHAEL L. MC CONNELL.** *Analytical Chemistry,* 1981, vol. 53 (8), 1007 A